(19) Europäisches Patentamt / European Patent Office / Office européen des brevets

(11) EP 4 260 813 A1

(12) EUROPEAN PATENT APPLICATION
published in accordance with Art. 153(4) EPC

(43) Date of publication:
18.10.2023 Bulletin 2023/42

(21) Application number: 21903086.3

(22) Date of filing: 09.11.2021

(51) International Patent Classification (IPC):
A61B 8/08 (2006.01)          A61B 5/22 (2006.01)

(52) Cooperative Patent Classification (CPC):
A61B 5/22; A61B 8/08

(86) International application number:
PCT/JP2021/041134

(87) International publication number:
WO 2022/123982 (16.06.2022 Gazette 2022/24)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 09.12.2020 JP 2020203909

(71) Applicant: Furuno Electric Co., Ltd.
Nishinomiya-City, Hyogo 662-8580 (JP)

(72) Inventor: KIYAN, Wataru
Nishinomiya-City, Hyogo 6628580 (JP)

(74) Representative: Müller Hoffmann & Partner
Patentanwälte mbB
St.-Martin-Strasse 58
81541 München (DE)

(54) ANALYSIS SYSTEM, ANALYSIS METHOD, ANALYSIS DEVICE, AND ANALYSIS PROGRAM

(57) [Problem] To provide an analysis system which can accurately and conveniently analyze a function of a muscle. [Solution] This analysis system 1 comprises: an electric stimulus device 2 which applies an electric stimulus formed of short pulses on a muscle; an ultrasonic wave pulse echo device 3 which transmits an ultrasonic wave to the muscle and receives a reflected ultrasonic wave; and an analysis unit 33 which analyzes, on the basis of the reflected ultrasonic wave, contraction characteristics of the muscle at the time of a simple contraction response of the muscle with respect to the electric stimulus.

FIG. 1

EP 4 260 813 A1

**Description**

Technical Field

The disclosure relates to an analysis system, an analysis method, an analysis device, and an analysis program for analyzing changes in a muscle caused by an electric stimulus using an ultrasonic wave.

Conventional Art

Technologies involving applying an electric stimulus to a muscle and analyzing changes of the muscle caused by the electric stimulus by using ultrasonic waves (for example, see Patent Documents 1 and 2) have been proposed.

Patent Document 1 discloses a training apparatus in which an ultrasonic wave probe is assembled to an electric stimulus (EMS) device. Accordingly, the user can easily confirm the thickness of the muscle and subcutaneous fat before and after training, as well as the movement of the muscle during training through an echo image.

Patent Document 2 discloses an ultrasonic wave diagnose device that includes an ultrasonic wave probe and EMS electrodes arranged around the ultrasonic wave probe. Electric stimuli are applied to a muscle by using the EMS electrodes, and the deformation (stretching speed, stretching acceleration, and elongation) of the muscle caused by the electric stimulus is measured by using the EMS. By applying the electric stimuli to the muscle and measuring the deformation of the muscle not affected by the environment or physical condition, it is possible to predict the development of the muscle.

Citation List

Patent Literature

Patent Document 1: PCT International Publication No. 2018/147384

Patent Document 2: Japanese Patent No. 5159326

SUMMARY

Technical Problem

In Patent Document 1, changes of muscle thickness, etc., in a long-term perspective (comparison before and after training, confirmation of training effects) are detected, but Patent Document 1 does not mention instant changes of muscle thickness in response to an electric stimulus (muscle contraction response). In other words, Patent Document 1 merely quantifies the static muscle morphology information (thickness), and is unable to obtain indicators related to muscle functions.

In Patent Document 2, muscle contraction characteristics vary with input conditions such as the transmission cycle of electric stimuli, the skin condition (moisture, sebum content, impedance, etc.), and muscle positions (depth). Therefore, in order to more accurately analyze the function of the muscle, the input conditions of the electric stimuli need to be defined. However, Patent Document 2 does not specifically mention the input conditions of the electric stimuli, and it is unclear on which kind of distortion at the time of the electric stimuli Patent Document 2 focuses.

A purpose of this disclosure is to solving the issues mentioned above, and the disclosure aims to provide an analysis system capable of accurately and easily analyzing the function of the muscle.

Solution to Problem

An analysis system according to the disclosure includes: an electric stimulus device that applies the electric stimulus formed by short pulses to the muscle; an ultrasonic wave pulse echo device that transmits ultrasonic waves to the muscle and receives reflected ultrasonic waves; and an analysis unit that analyzes a contraction characteristic of the muscle at a time of a single contraction response of the muscle to the electric stimulus based on the reflected ultrasonic waves.

An analysis method according to the disclosure includes: applying an electric stimulus formed by short pulses to a muscle, transmitting ultrasonic waves to the muscle to receive reflected ultrasonic waves, and analyzing a contraction characteristic of the muscle at a time of a single contraction response of the muscle to the electric stimulus based on the reflected ultrasonic wave.

An analysis device according to the disclosure analyzes a contraction characteristic of the muscle at a time of a single contraction response of the muscle to an electric stimulus formed by short pulses applied to the muscle based on ultrasonic waves transmitted to the muscle and reflected.

An analysis program according to the disclosure causes a computer to operate as an analysis device to analyze a contraction characteristic of a muscle at a time of a single contraction response of the muscle to an electric stimulus formed by short pulses applied to the muscle based on ultrasonic waves transmitted to the muscle and reflected.

Effects of Disclosure

According to the disclosure, it is possible to analyze the function of the muscle accurately and easily.

BRIEF DESCRIPTION OF DRAWINGS

FIG. 1 is a block diagram of an analysis system according to an embodiment of the disclosure.

FIG. 2 is a schematic diagram of the analysis system according to an embodiment of the disclosure.

FIG. 3 is a flowchart illustrating a processing flow of an analysis method according to an embodiment of the disclosure.

FIG. 4 is a flowchart illustrating the processing flow of the analysis method according to an embodiment of the disclosure.

In FIG. 5, (A) of FIG. 5 is an example of an M-mode image, (B) of FIG. 5 is a waveform showing a displacement of a muscle boundary in a region of interest (ROI) of the M-mode image shown in (A) of FIG. 5, and (C) of FIG. 5 is a waveform showing a part of a displacement waveform in (B) of FIG. 5 after a normalization process.

FIG. 6 is a diagram explaining a method for observing a single contraction response using a B-mode ultrasonic wave.

DESCRIPTION OF EMBODIMENTS

The embodiments of the disclosure are described in the following with reference to the accompanying drawings. However, the disclosure is not limited to the following embodiments.

(System Configuration)

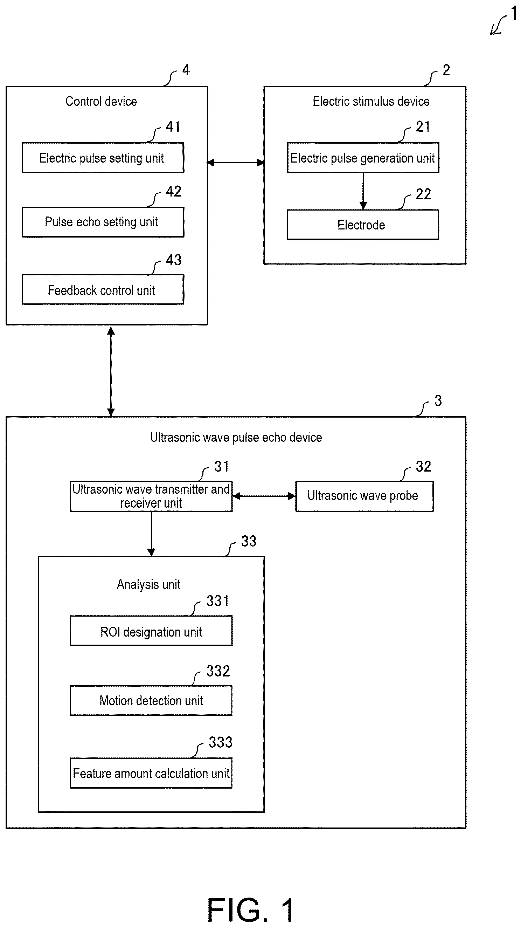

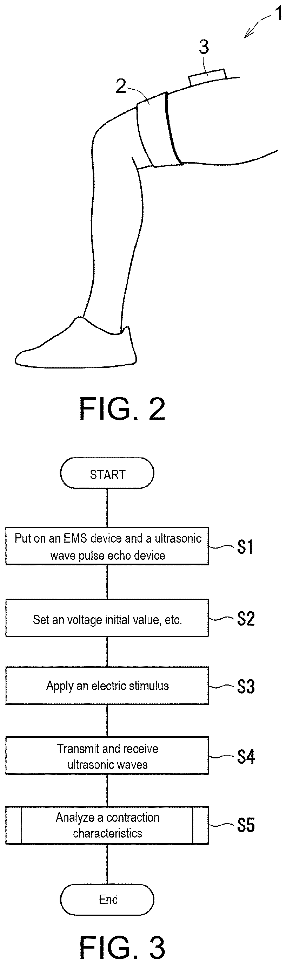

FIG. 1 is a block diagram of an analysis system 1 according to an embodiment of the disclosure, and FIG. 2 is a schematic diagram of the analysis system 1. The analysis system 1 includes an electric stimulus device (e.g., an electrical muscle stimulation (EMS) device) 2, an ultrasonic wave pulse echo device 3, and a control device 4. In FIG. 2, the illustration of the control device 4 is omitted.

The electric stimulus device 2 is a device that applies an electric stimulus to human tissues such as a muscle, and includes an electric pulse generation unit 21 and electrodes 22. The shape of the electric stimulus device 2 is not particularly limited, but in the embodiment, the electric stimulus device 2 is belt-shaped, as shown in FIG. 2, and the electrodes 22 are installed to contact the skin surface of the leg (in the embodiment, the thigh). The electric pulses generated by the electric pulse generation unit 21 propagate through the electrodes 22 into the human body. When the electric pulses reach the muscle, the muscle contracts in response to the electric stimulus.

The ultrasonic wave pulse echo device 3 serves to transmit ultrasonic waves to the muscle and receive reflected ultrasonic waves. As shown in FIG. 1, the ultrasonic wave pulse echo device 3 includes an ultrasonic wave transmitter and receiver unit 31, an ultrasonic wave probe 32, and an analysis unit 33. In the embodiment, as shown in FIG. 2, the ultrasonic wave pulse echo device 3 is attached to the skin surface of the thigh. The ultrasonic wave transmitter and receiver unit 31 transmits ultrasonic waves towards the inside of the human body through the ultrasonic wave probe 32 in contact with the skin surface and receives the ultrasonic waves reflected inside the human body. In the embodiment, in order to confirm the evaluation site, two modes of the ultrasonic wave are used: ultrasonic waves for imaging (B-mode) and ultrasonic waves for motion detection (M-mode) for quantitative evaluation of muscles. However, it is acceptable to transmit only the motion detection ultrasonic waves. Alternatively, it may also be that only the ultrasonic waves for imaging (B-mode) with a high frame rate capable of motion detection is used.

The analysis unit 33 includes an analysis device serving to analyze the muscle function based on the ultrasonic waves transmitted and received by the ultrasonic wave pulse echo device 3. For this purpose, in the embodiment, the analysis unit 33 includes an ROI (also referred to as "region of interest" in the following) designation unit 331, a motion detection unit 332, and a feature amount calculation unit 333. These functional blocks may be realized as hardware by using logical circuits formed on an integrated circuit, or realized as software by deploying a particular program in a memory and executing the program using the processor of the ultrasonic wave pulse echo device 3.

The control device 4 is a device that controls the electric stimulus device 2 and the ultrasonic wave pulse echo device 3, and may be configured by using a general-purpose computer, for example. As shown in FIG. 1, the control device 4 includes an electric pulse setting unit 41, a pulse echo setting unit 42, and a feedback control unit 43.

The electric pulse setting unit 41 serves to set the pulse width, repetition frequency, voltage, and the like of the electric pulses generated by the electric pulse generation unit 21 of the electric stimulus device 2. The pulse width is set to be sufficiently smaller (e.g., 1 ms) than a single contraction time of the muscle (on the order of up to 100 ms). The repetition frequency of the electric pulses is set to be a pulse interval (e.g., 2 Hz) that allows sufficient observation of a single contraction response of the muscle (one muscle contraction response when a short-pulse electric stimulus is applied to the muscle).

Here, a short pulse is defined as an electrical pulse wave of 10 ms or less, which is sufficiently shorter than the single contraction response time of the muscle (on the order of up to 100 ms). A pulse width of 1 ms or less (for example, one cycle of a square wave with a fundamental wave frequency of 2 kHz (pulse width: 0.5 ms, etc.)) may be adopted, but even 20 cycles of a square burst wave with a fundamental wave frequency of 2 kHz (pulse width: 10 ms) are defined as a short pulse here. The voltage of the electric pulse is initially set by the user's operation, and while the electric stimulus device 2 is applying the electric stimulus to the muscle, it is reset according to the control of the feedback control unit 43.

The pulse echo setting unit 42 serves to set the waveform, the transmission center frequency, the transmission repetition period (frame rate), the transmission pulse repetition frequency, and the sampling frequency of the ultrasonic waves transmitted by the ultrasonic wave transmitter and receiver unit 31 of the ultrasonic wave pulse echo device 3. For example, the ultrasonic waves for imaging (B-mode) are set with a transmission center frequency of 5 MHz and a frame rate of 30 Hz. The ultrasonic waves for motion detection (M-mode) are set with a transmission center frequency of 10 MHz and a transmission pulse repetition frequency of 2 kHz.

In the case of a system with which a sufficient repetition frequency cannot be obtained for observing the muscle contraction responses even in the M-mode, it is effective to lower the frame rate of the ultrasonic waves in the B-mode and prioritize the repetition frequency of the ultrasonic waves in the M-mode and set repetition frequency to a high value. At this time, it is possible to achieve both functions without interference by separating the transmission frequency band for imaging and the transmission frequency band for motion detection and separating the respective signals through a filtering process at the time of reception.

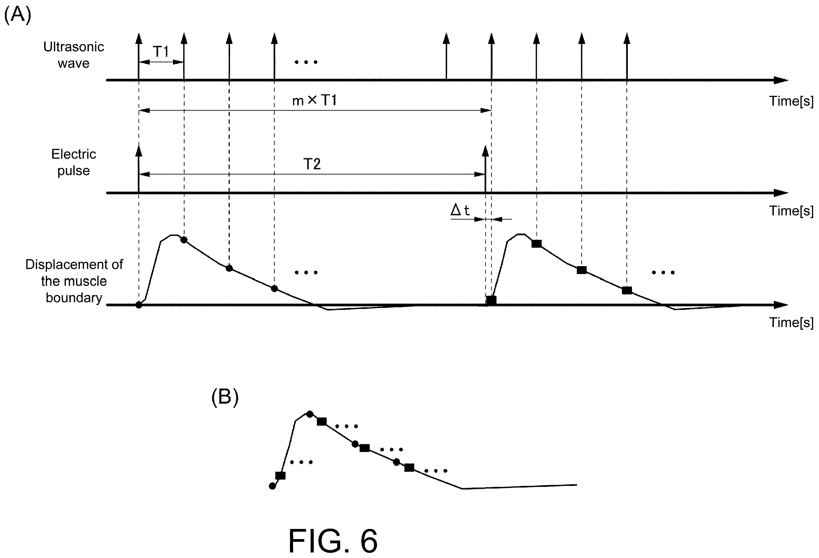

Furthermore, even in the case of the normal ultrasonic waves of the B-mode with a low frame rate, it is possible to observe the single contraction response by slightly shifting the repetition period of the electric pulses with respect to an integer multiple of an ultrasonic wave frame rate and under-sampling. For example, as shown in (A) of FIG. 6, if the ultrasonic wave frame rate is T1 and the repetition period of the electric pulse (short pulse) is T2, then the following is set:

$$T2 = m \times T1 - \triangle t$$

$$\triangle t = T1 / n,$$

where m and n are particular integers. By setting T1 = 1/30 s, m = 15, n = 60 ($\triangle t$ = 1/1800 s), it is possible to observe one single contraction response by ultrasonic imaging the response to 60 electric pulses. That is, by sampling the displacement of a muscle boundary detected by the ultrasonic waves in a time frame of each period of the electric pulse and overlapping the displacements of the muscle boundary in 60 time frames in one time frame, it is possible to estimate the displacement of the muscle boundary during the single contraction response, as shown in (B) of FIG. 6.

The sampling method above merely serves as an example, and it is also possible to set the transmission timing of electric pulse to fall slightly behind the sampling point of ultrasonic wave imaging by setting T2 = m × T1 + $\triangle t$. Furthermore, a setting where the waveform obtained by overlapping is sampled at equal intervals is described, but it is also possible to estimate the displacement of the muscle boundary during the single contraction response, which is the goal, even with a setting where $\triangle t$ is not an integer division of T1.

The feedback control unit 43 serves to control the magnitude of the electric stimulus in response to the muscle contraction. In the embodiment, the feedback control unit 43 controls the electric pulse setting unit 41 to reset the voltage of the electric pulse generated by the electric pulse generation unit 21 in response to the magnitude of the muscle contraction as detected by the motion detection unit 332 of the analysis unit 33 when the electric stimulus is applied.

The electric stimulus device 2, the ultrasonic wave pulse echo device 3, and the control device 4 may also be integrated to form the analysis system 1 as a single device. Additionally, the analysis unit 33 may also be configured as a separate analysis device from the ultrasonic wave pulse echo device 3, and the control device 4 may also be provided with the function of the analysis unit 33.

(Processing Procedure)

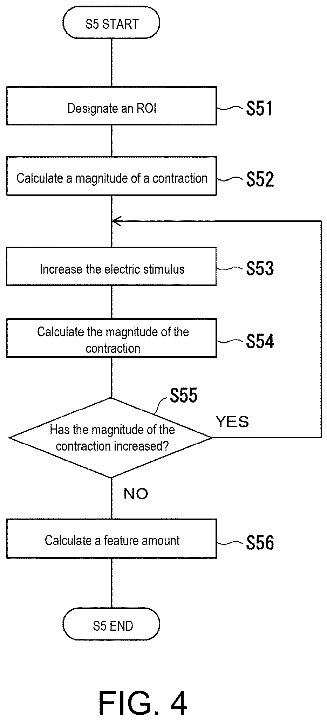

FIG. 3 and FIG. 4 are flowcharts illustrating processing steps in an analysis method according to the embodiment. The analysis method according to the embodiment can be carried out by the analysis system 1 shown in FIG. 1.

In Step S1, the electric stimulus device 2 and the ultrasonic wave pulse echo device 3 are attached to the human body. In the embodiment, as shown in FIG. 2, the electric stimulus device 2 and the ultrasonic wave pulse echo device 3 are attached to the thigh.

In Step S2, the electric pulse setting unit 41 sets the initial voltage value of the electric pulses generated by the electric pulse generation unit 21. The initial voltage value may be appropriately changed depending on the type, size, and position (depth) of the muscle. The electric pulse setting unit 41 may further set the pulse width or the repetition frequency of the electric pulses as needed. In addition, if the initial voltage value is set in advance, Step S2 may be omitted.

In Step S3, the electric pulse generation unit 21 generates electric pulses to start applying the electric stimulus to the muscle via the electrodes 22. In the embodiment, the repetition frequency of the electric pulses is 2 Hz. When the electric stimulus is applied to the muscle, the boundary surface of the muscle displaces in a direction perpendicular to the longitudinal direction (direction of muscle fibers) of the muscle due to muscle contraction.

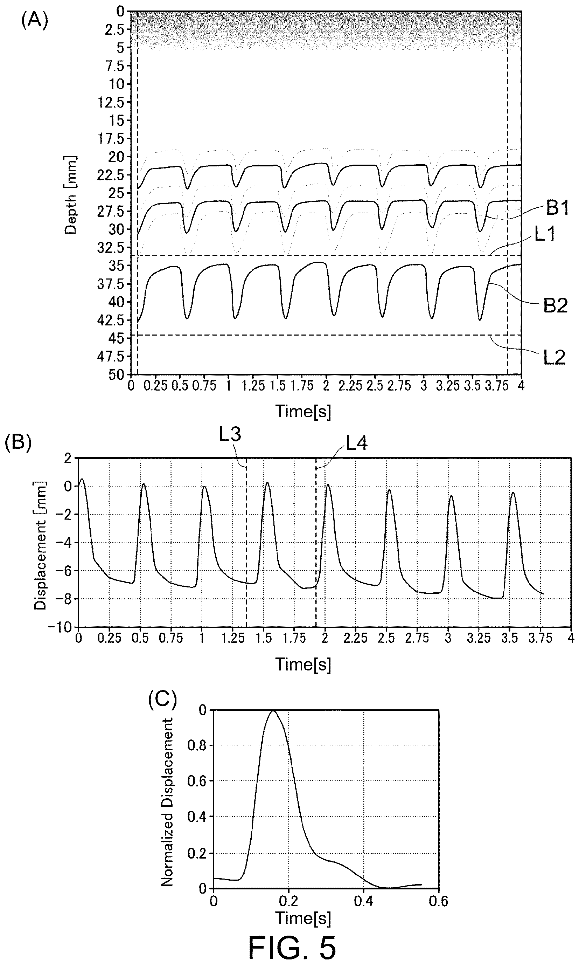

In Step S4, the ultrasonic wave transmitter and receiver unit 31 starts transmitting and receiving ultrasonic waves through the ultrasonic wave probe 32. The transmitted ultrasonic waves are reflected at the boundary surface of the muscle (the boundary surface of the muscle with another muscle, the boundary surface between the muscle and soft tissues, the boundary surface between the muscle and a bone), and the reflected ultrasonic waves are received by the ultrasonic wave transmitter and receiver unit 31 through the ultrasonic wave probe 32. Based on the time lag of the transmission and reception of the ultrasonic waves, an M-mode image showing the motion of the muscle is obtained. An example of the M-mode image is shown in (A) of FIG. 5. The M-mode image contains peaks with a cycle of 0.5s corresponding to each muscle contraction.

The order of Steps S3 and S4 is not specifically limited.

In Step S5, the analysis unit 33 analyzes the contraction characteristic of the muscle. FIG. 4 shows the detailed procedure of Step S5.

In Step S51, the ROI designation unit 331 sets the ROI in the M-mode image shown in (A) of FIG. 5. A waveform B1 in (A) of FIG. 5 corresponds to the boundary between a vastus intermedius muscle and a rectus femoris muscle, and a waveform B2 corresponds to the boundary between the vastus intermedius muscle and a femur. In the embodiment, the ROI designation unit 331 sets the region between lines L1 and L2 corresponding to the vastus intermedius muscle, which is the analysis target, as the ROI. The ROI may also be set manually by the user with reference to an echo tomographic image, or may also be set automatically.

In Step S52, the motion detection unit 332 extracts the waveform indicating the displacement of the muscle boundary in the ROI through a tracking process, and detects the magnitude of the contraction of the muscles (vastus intermedius and rectus femoris) based on the waveform. (B) of FIG. 5 shows the waveform indicating the displacement of the muscle boundary that is extracted. In the embodiment, the magnitude of contraction indicates the displacement amount of the boundary surface of the muscle in a particular direction perpendicular to the longitudinal direction of the muscle. The motion detection unit 332 calculates the difference between the maximum and the minimum of the waveform (for example, the waveform between lines L3 and L4) corresponding to one contraction as the magnitude of contraction. The calculated magnitude of contraction is transmitted to the feedback control unit 43 of the control device 4.

In the embodiment, an example of analyzing the muscle contraction response in both the rectus femoris muscle and the vastus intermedius muscle is shown. However, if it is desired to separately extract the single contraction characteristic of the vastus intermedius muscle, such single contraction characteristic may be extracted by extracting the time response of the displacement of each of the waveforms B1 and B2 shown in (A) of FIG. 5 through the tracking process, and analyzing the displacement difference.

In Step S53, the feedback control unit 43 controls the magnitude of the electric stimulus based on the magnitude of the muscle contraction. In the embodiment, the feedback control unit 43 controls the electric pulse setting unit 41 to increase the electric stimulus until the maximum contraction of the single muscle contraction saturated (referring to a state where the muscle contracts at an intensity at which the intensity of the electric stimulus formed by short pulses maximizes the contraction of the muscle). When performing Step S7 for the first time, the feedback control unit 43 causes the electric pulse setting unit 41 to reset the voltage, so that the voltage of the electric pulse is greater than the initial vale by a particular value.

In Step S54, in the same manner as Step S52, the motion detection unit 332 recalculates the magnitude of the muscle contraction. If the magnitude of the muscle contraction is greater than the magnitude before the electric stimulus is increased (YES in Step S55), the process returns to Step S53, and the feedback control unit 43 resets the voltage of the electric pulse setting unit 41, so as to further increase the voltage of the electric pulse by a particular value.

Alternatively, in the case where the magnitude of the muscle contraction does not change compared with the magnitude before the electric stimulus is increased (NO in Step S55), the feedback control unit 43 maintains the voltage of the electric pulse without instructing the electric pulse setting unit 41 to reset the voltage.

In the embodiment, the state where, even if the electric stimulus is increased, the magnitude of the maximum contraction of the muscle does not substantially change before and after the increase is defined as the state where the muscle contraction is saturated. There is no specific limitation on the criteria for determining whether the maximum contraction of the muscle has been saturated, but the muscle contraction may be determined as having been saturated if the increase rate of the contraction after the electric stimulus is increased is less than or equal to a particular value (for example, 2%) compared to the contraction before the electric stimulus is increased.

Then, the process proceeds to Step S56. The feature amount calculation unit 333 calculates a feature amount that represents the contraction characteristic of the muscle when the muscle contraction in response to the electric stimulus has been saturated (hereinafter referred to as saturated contraction). In the embodiment, the feature amount calculation unit 333 calculates, as the feature amount, a half-width of the peak corresponding to the muscle contraction in the waveform indicating the displacement.

Specifically, the feature amount calculation unit 333 extracts the waveform (for example, the waveform between lines L3 and L4 shown in (B) of FIG. 5) that indicates the displacement during one contraction, and performs a normalization process so that the maximum of the displacement amount is 1 and the minimum is 0. The waveform after the normalization process is shown in (C) of FIG. 5. The feature amount calculation unit 333 calculates the time when the displacement amount is 0.5 or more in the waveform as the half width.

The calculated half-width may be an indicator of the muscle function, as described below. For example, when comparing the intermediate vastus muscle and the soleus muscle of the same person, the soleus muscle tends to have a larger half-width than the intermediate vastus muscle because the soleus muscle is more of a slow-twitch-muscle-dominant muscle than the intermediate vastus muscle. Even for the same type of muscle, as will be described below, subjects with diseases such as sarcopenia have different half-widths from healthy individuals, due to loss of fast-twitch muscle fibers or infiltration of fat between muscle fibers.

The feature amount is not limited to half-width as long as the feature amount represents the contraction characteristic of the muscle. For example, the feature amount may be the 10% width of the peak, or the rise or fall time of the peak. Additionally, the analysis method of the contraction characteristic of the muscle is not limited to the calculation of the feature amount, and may also be a determination on whether the function of the muscle is good or poor.

(Overview)

In the embodiment, the contraction characteristic of the muscle when the contraction of the muscle is saturated, i.e., when the magnitude of the muscle contraction does not substantially change even if the electric stimulus is increased, is analyzed. Since the contraction characteristic when the contraction is saturated is not influenced by the will of the subject and does not depend on the input conditions of the electric stimulus, such contraction characteristic objectively reflects the function of the muscle. Therefore, the muscle function can be accurately and easily analyzed without using a CT or MRI device. Comparatively, in the conventional technology described in Patent Document 2, the input conditions of the electric stimulus are not disclosed, and it is unclear whether the muscle function can be accurately detected.

The above describes embodiments of the disclosure. However, the disclosure is not limited to the above embodiments and various modifications are possible without departing from the scope of the disclosure.

[Industrial Applicability]

This disclosure, for example, is applicable for prevention of sarcopenia and for supporting training for athletes.

To prevent sarcopenia, it is known that the initial symptoms of sarcopenia include loss of fast-twitch muscle fibers and infiltration of fat between muscle fibers. As sarcopenia progresses, the muscle cross-sectional area further decreases, leading to a significant decrease in QOL, such as difficulty in walking. Therefore, according to the disclosure, the progression of sarcopenia can be prevented by measuring the contraction characteristic of the muscle at a particular interval (for example, every six months), and detecting early sarcopenic changes, such as the loss of fast-twitch muscle fibers and infiltration of fat into muscle fibers, based on the temporal changes, before a decrease in the muscle cross-sectional area is observed.

In addition, in athlete training, it is important for performance improvement to grasp the muscle composition (ratio between fast-twitch muscles and slow-twitch muscles) suitable for the event and to know whether the muscles are changing with a desired tendency as a result of training. Therefore, according to the disclosure, it is possible to objectively and easily determine the effect of training by measuring the ratio between fast-twitch muscles and slow-twitch muscles, etc.

Reference Signs List

1. Analysis system
2. Electric stimulus device
3. Ultrasonic wave pulse echo device
4. Control device
21. Electric pulse generation unit
22. Electrode
31. Ultrasonic wave transmitter and receiver unit
32. Ultrasonic wave probe
33. Analysis unit
331. ROI designation unit
332. Motion detection unit
333. Feature amount calculation unit
41. Electric pulse setting unit
42. Pulse echo setting unit
43. Feedback control unit

**Claims**

1. An analysis system, comprising:

   an electric stimulus device that applies an electric stimulus formed by short pulses to a muscle;
   an ultrasonic wave pulse echo device that transmits ultrasonic waves to the muscle and receives reflected ultrasonic waves; and
   an analysis unit that analyzes a contraction characteristic of the muscle at a time of a single contraction response of the muscle to the electric stimulus based on the reflected ultrasonic waves.

2. The analysis system according to claim 1, wherein the electric stimulus is formed by the short pulses with a particular repetition frequency.

3. The analysis system according to claim 1 or 2, wherein the analysis unit calculates a feature amount indicating the contraction characteristic.

4. The analysis system according to claim 3, wherein the analysis unit calculates, as the feature amount, a half-width of a peak corresponding to the single contraction in a waveform that represents the single contraction response of the muscle.

5. The analysis system according to any one of claims 1 to 4, further comprising a feedback control unit that controls a magnitude of the electric stimulus in response to a magnitude of the single contraction of the muscle.

6. The analysis system according to claim 5, wherein the feedback control unit increases the electric stimulus until the single contraction response of the muscle is saturated.

7. The analysis system according to any one of claims 1 to 6, wherein a repetition period of the short pulses is shifted by a particular time from an integer multiple of a transmission repetition period of the ultrasonic waves transmitted by the ultrasonic wave pulse echo device, and the particular time is shorter than the transmission repetition period of the ultrasonic waves, and
the analysis unit estimates a displacement of a muscle boundary of the single contraction response by sampling the displacement of the muscle boundary detected by the ultrasonic waves in a time frame of each period of the short pulses and overlapping the displacements of the muscle boundary in a plurality of time frames into one time frame.

8. An analysis method, comprising:

applying an electric stimulus formed by short pulses to a muscle,
transmitting ultrasonic waves to the muscle to receive reflected ultrasonic waves, and
based on the reflected ultrasonic waves, analyzing a contraction characteristic of the muscle at a time of a single contraction response of the muscle to the electric stimulus.

9. An analysis device, analyzing a contraction characteristic of a muscle at a time of a single contraction response of the muscle to an electric stimulus formed by short pulses applied to the muscle based on ultrasonic waves transmitted to the muscle and reflected.

10. An analysis program, causing a computer to operate as an analysis device to analyze a contraction characteristic of a muscle at a time of a single contraction response of the muscle to an electric stimulus formed by short pulses applied to the muscle based on ultrasonic waves transmitted to the muscle and reflected.

FIG. 1

FIG. 2

```
        ┌──────────┐
        │  START   │
        └──────────┘
             │
┌────────────────────────────┐
│ Put on an EMS device and a  │── S1
│ ultrasonic wave pulse echo  │
│          device             │
└────────────────────────────┘
             │
┌────────────────────────────┐
│ Set an voltage initial      │── S2
│ value, etc.                 │
└────────────────────────────┘
             │
┌────────────────────────────┐
│  Apply an electric stimulus │── S3
└────────────────────────────┘
             │
┌────────────────────────────┐
│   Transmit and receive      │── S4
│   ultrasonic waves          │
└────────────────────────────┘
             │
┌────────────────────────────┐
│  Analyze a contraction      │── S5
│  characteristics            │
└────────────────────────────┘
             │
        ┌──────────┐
        │   End    │
        └──────────┘
```

FIG. 3

```
         ┌──────────────┐
         │   S5 START    │
         └──────┬───────┘
                │
         ┌──────┴───────────────┐
         │   Designate an ROI   │─── S51
         └──────┬───────────────┘
                │
   ┌────────────┴──────────────────────┐
   │ Calculate a magnitude of a        │─── S52
   │ contraction                       │
   └────────────┬──────────────────────┘
                │
                ↓←──────────────────────────┐
   ┌────────────┴──────────────────────┐    │
   │  Increase the electric stimulus   │─── S53
   └────────────┬──────────────────────┘    │
                │                            │
   ┌────────────┴──────────────────────┐    │
   │  Calculate the magnitude of the   │─── S54
   │           contraction             │    │
   └────────────┬──────────────────────┘    │
                │          S55               │
             ╱──┴──╲                         │
          ╱           ╲        YES           │
        ╱  Has the      ╲────────────────────┘
        ╲  magnitude of  ╱
          ╲ the         ╱
          ╱ contraction
          ╲ increased? ╱
             ╲──┬──╱
                │  NO
   ┌────────────┴──────────────────────┐
   │   Calculate a feature amount      │─── S56
   └────────────┬──────────────────────┘
                │
         ┌──────┴───────┐
         │   S5 END      │
         └──────────────┘
```

# FIG. 4

FIG. 5

(A)

Ultrasonic wave

T1

m × T1

Time[s]

Electric pulse

T2

Time[s]

Δt

Displacement of the muscle boundary

Time[s]

(B)

FIG. 6

EP 4 260 813 A1

## INTERNATIONAL SEARCH REPORT

| International application No. |
|---|
| **PCT/JP2021/041134** |

**A. CLASSIFICATION OF SUBJECT MATTER**

*A61B 8/08*(2006.01)i; *A61B 5/22*(2006.01)i
FI: A61B8/08; A61B5/22 200

According to International Patent Classification (IPC) or to both national classification and IPC

**B. FIELDS SEARCHED**

Minimum documentation searched (classification system followed by classification symbols)

A61B8/00 - 8/15; A61B5/06 - 5/22; A61H1/00 - A61H5/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | WO 2014/174379 A1 (AMID S.R.L.) 30 October 2014 (2014-10-30) p. 8, line 27 to p. 14, line 13, fig. 1-7 | 1-3, 8-10 |
| A | p. 8, line 27 to p. 14, line 13, fig. 1-7 | 4-7 |
| A | JP 2017-006632 A (PANASONIC INTELLECTUAL PROPERTY MANAGEMENT CO., LTD.) 12 January 2017 (2017-01-12) paragraphs [0010]-[0193], fig. 1-21 | 1-10 |
| A | JP 2019-150544 A (MTG KK) 12 September 2019 (2019-09-12) paragraph [0027], fig. 4 | 1-10 |

☐ Further documents are listed in the continuation of Box C.    ☑ See patent family annex.

| | | |
|---|---|---|
| * Special categories of cited documents: | "T" | later document published after the international filing date or priority date and not in conflict with the application but cited to understand the principle or theory underlying the invention |
| "A" document defining the general state of the art which is not considered to be of particular relevance | | |
| "E" earlier application or patent but published on or after the international filing date | "X" | document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" document referring to an oral disclosure, use, exhibition or other means | | |
| "P" document published prior to the international filing date but later than the priority date claimed | "&" | document member of the same patent family |

| Date of the actual completion of the international search | Date of mailing of the international search report |
|---|---|
| **04 January 2022** | **18 January 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2021/041134**

| Patent document cited in search report | | | Publication date (day/month/year) | Patent family member(s) | | | Publication date (day/month/year) |
|---|---|---|---|---|---|---|---|
| WO | 2014/174379 | A1 | 30 October 2014 | US 2016/0095575 A1 paragraphs [0043]-[0067], fig. 1-7 | | | |
| JP | 2017-006632 | A | 12 January 2017 | (Family: none) | | | |
| JP | 2019-150544 | A | 12 September 2019 | WO 2019/172297 | A1 | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018147384 W

- JP 5159326 B